# EUROPEAN PATENT APPLICATION

(11) **EP 4 306 110 A1**
(43) Date of publication of application: **17.01.2024**
(21) Application number: 22767339.9
(22) Date of filing: 07.02.2022
(51) Int. Cl.: A61K 31/44, A61K 31/352, A61P 3/00, A61P 1/16, A23L 33/10

(54) **COMPOSITION FOR PREVENTING OR TREATING METABOLIC DISEASES, CONTAINING TORSEMIDE AND CROMOLYN**

(30) Priority: 08.03.2021 KR 20210029950
(71) Applicant: Oncocross Co., Ltd., Seoul 04168 (KR)
(72) Inventor: KIM, Yi-Rang, Sejong 30064 (KR)
(74) Representative: Isarpatent
(86) International application number: PCT/KR2022/001844
(87) International publication number: WO 2022/191443

(57) **Abstract**

The present invention relates to a composition for the treatment of metabolic diseases, containing torsemide and cromolyn. According to the present invention, if torsemide and cromolyn are used in combination, compared to when cromolyn alone is used, weight is reduced, insulin resistance is reduced and liver fat accumulation is remarkably reduced in a NASH animal model, and thus the present invention can be effectively used in the prevention or treatment of metabolic diseases including diabetes, obesity, insulin resistance and metabolic liver diseases.

## Description

### [Technical Field]

The present invention relates to a composition for treating metabolic diseases containing torsemide and cromolyn.

### [Background Art]

The liver is a large organ located in the upper abdominal cavity on the right side of the body and weighs about 1.5 Kg in adults. A normal liver visually has a reddish color and has a smooth surface. The shape and size of the liver change when disease occurs in the liver. As a representative example, when fat is accumulated in the liver due to obesity or heavy drinking etc., the size of the liver increases and the surface of liver changes into a yellow color. Conversely, as cirrhosis worsens, the size of the liver decreases and the surface becomes bumpy. The liver is a very important organ with many functions, and the functions are summarized as follows. First, the liver has a function of properly processing various nutrients in our body, that is, a metabolic function. The foods absorbed from the intestines are properly changed in the liver so as to be used in various tissues of the body, and the waste products left after using nutrients in various tissues are transferred back to the liver to be processed. Second, the liver stores some nutrients required for our body. Third, the liver makes bile acid, which is an essential substance for nutrient absorption in the intestine, and discharge the bile acid into the intestine through the biliary tract. If these bile acids are not produced, many nutrients are not absorbed, resulting in nutritional deficiencies. Fourth, the liver has a function of making substances (albumin, blood clotting protein, cholesterol, etc.) that are absolutely necessary for our body to function properly. Finally, the liver has a function of detoxifying alcohol (alcohol), drugs, and various toxins occurring from our body (Eugene Brainward, et al. Harrison's principles of internal medicine. 15th edition. 2001).

Non-alcoholic fatty liver disease (NAFLD) is defined as an accumulation of 5% or more of fatty acids in the parenchymal cells of the liver in the form of triglycerides unlike alcohol-induced liver damage. Pathologically, the NAFLD is classified into simple steatosis and steatohepatitis with inflammation. Non-alcoholic steatohepatitis (NASH) is a disease that occurs during the exacerbation of the NAFLD, and has been recognized as a serious disease worldwide because as fat accumulates in hepatocytes, hepatocyte degeneration/necrosis occurs to be developed into cirrhosis and liver cancer by inflammation and liver fibrosis. The NASH has been suggested that the direct induction of liver damage along with intrahepatic fat accumulation by excessive free fatty acid (FFA) plays an important role in the body. In the case of obesity and insulin resistance, the influx of FFA into the liver increases, but the FFA accumulates fat in the liver through β-oxidation or esterification of triglycerides (TG). In addition, direct liver damage occurs due to an increase in oxidative stress and activation of inflammatory signaling process due to excessive FFA itself, and TG accumulates in the liver as a protective mechanism for the liver damage. As a result, hepatocyte death and insufficient repair ultimately lead to NASH, a critical lesion that causes liver fibrosis and disease progression (Ong JP et al., Obesity Surgery volume 15, pages 310-315, 2005). The NASH is divided into primary NASH and secondary NASH depending on the cause, and the primary NASH is caused by hyperlipidemia, diabetes, or obesity, which are characteristics of metabolic syndrome (Szczepaniak LS et al., 2005), and the secondary NASH is known to be caused by nutritional causes (rapid weight loss, starvation, intestinal bypass), various drugs, toxic substances (poisonous mushrooms, bacterial toxins), metabolic causes, and other factors. It has been known that the incidence of nonalcoholic steatohepatitis related to diabetes and obesity, which is an important feature of metabolic syndrome, which is a primary factor, occurs in about 50% of diabetic patients, about 76% of obese patients, and almost all of obese diabetic patients (Gupte P et al., 2004). In addition, biopsies performed in diabetic and obese patients with increased levels of alanine aminotransferase (ALT) showed that the incidence of steatohepatitis was 18 to 36% (Braillon A et al., 1985).

The NASH has been considered a benign disease that does not progress, but in spite of non-drinkers, the NASH has been found to show inflammation patterns similar to alcoholic hepatitis and liver fibrosis, and known to be a disease with a poor prognosis. In particular, in recent years, metabolic syndrome caused by obesity, diabetes, etc. have received attention and the idea that nonalcoholic steatohepatitis (NASH) is one of these syndromes is spreading. However, the mechanism is unclear, and no effective treatment or therapeutic agent has been established. To date, there is no established treatment for NASH, which is because NASH is associated with various factors such as diabetes, obesity, coronary artery disease, and lifestyle. Effects on fatty liver diseases caused by the administration of several drugs for diabetes or obesity have been reported. It has been reported that Orlistat, which is used as an oral anti-obesity drug, induced histological improvement of the liver in patients with steatohepatitis (Hussein et al., 2007), and it has been reported that Metformin reduced blood liver enzyme levels, hepatic necrotic inflammation and fibrosis in patients with nonalcoholic steatohepatitis without diabetes (Bugianesi et al., 2005). In addition, it has been reported that a thiazolidinedione (TZD)-based drug, which is an agonist of peroxisome proliferator activated receptor (PPAR), inhibits fat accumulation in the liver and muscle, and has a direct anti-fibrotic action on the liver in an animal model of nonalcoholic steatohepatitis (Galli A et al., 2002). However, there is currently no composition that may be recognized as a drug treatment for nonalcoholic steatohepatitis, and it is urgent to develop a new therapeutic agent for the treatment or prevention of the disease.

### [Disclosure]

### [Technical Problem]

An object of the present invention is to provide a pharmaceutical composition for preventing or treating metabolic diseases.

Another object of the present invention is to provide a health functional food for preventing or improving metabolic diseases.

Yet another object of the present invention is to provide a method for treating metabolic diseases.

Yet another object of the present invention is to provide a use for use in the preparation of a pharmaceutical composition for preventing or treating metabolic diseases.

### [Technical Solution]

In order to achieve the object, an aspect of the present invention provides a pharmaceutical composition for preventing or treating metabolic diseases containing torsemide and cromolyn, or pharmaceutically acceptable salts thereof, as active ingredients.

Another aspect of the present invention provides a health functional food for preventing or improving metabolic diseases containing torsemide and cromolyn, or pharmaceutically acceptable salts thereof.

Yet another aspect of the present invention provides a method for treating metabolic diseases including administering torsemide and cromolyn, or pharmaceutically acceptable salts thereof in a pharmaceutically effective dose to a subject suffering from metabolic diseases.

Yet another aspect of the present invention provides a use of torsemide and cromolyn, or pharmaceutically acceptable salts thereof, for use in the preparation of a pharmaceutical composition for preventing or treating metabolic diseases.

### [Advantageous Effects]

According to the present invention, if torsemide and cromolyn are used in combination, compared to when cromolyn alone is used, weight is reduced, insulin resistance is reduced and liver fat accumulation is remarkably reduced in a NASH animal model, and thus the present invention can be effectively used in the prevention or treatment of metabolic diseases including diabetes, obesity, insulin resistance and metabolic liver diseases.

### [Description of Drawings]

FIG. 1 is a graph (average weight ± standard deviation) showing average weights (gram) of mice for each group measured for each week.
FIG. 2 is a diagram illustrating average fasting plasma glucose levels (mg/dl) of mice for each group measured at Week 13 of an experiment.
FIG. 3 is an image and a graph (staining % ± standard deviation) showing the degrees of neutral fat accumulation in liver tissue confirmed by Oil Red O staining.

### [Best Mode of the Invention]

Hereinafter, an exemplary embodiment of the present invention will be described in detail with reference to the accompanying drawings. However, the following exemplary embodiments are presented as examples for the present invention, and when it is determined that a detailed description of well-known technologies or configurations known to those skilled in the art may unnecessarily obscure the gist of the present invention, the detailed description thereof may be omitted, and the present invention is not limited thereto. Various modifications and applications of the present invention are possible within the description of claims to be described below and the equivalent scope interpreted therefrom.

Terminologies used in the present invention are terminologies used to properly express embodiments of the present invention, which may vary according to a user, an operator's intention, or customs in the art to which the present invention pertains. Therefore, these terminologies used in the present invention will be defined based on the contents throughout the specification. Throughout the specification, unless explicitly described to the contrary, when a certain part "comprises" a certain component, it will be understood to imply the inclusion of stated elements but not the exclusion of any other elements.

All technical terms used in the present invention, unless otherwise defined, have the meaning as commonly understood by those skilled in the related art of the present invention. In addition, although preferred methods and samples are described in the present invention, similar or equivalent methods and samples thereto are also included in the scope of the present invention. The contents of all publications disclosed as references in this specification are incorporated in the present invention.

In an aspect, the present invention provides a pharmaceutical composition for preventing or treating metabolic diseases containing torsemide and cromolyn, or pharmaceutically acceptable salts thereof, as active ingredients.

In an exemplary embodiment, the torsemide may be a compound represented by Chemical Formula 1 below, and the cromolyn may be a compound represented by Chemical Formula 2 below: 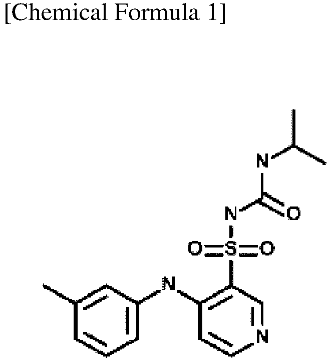 ; and

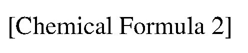

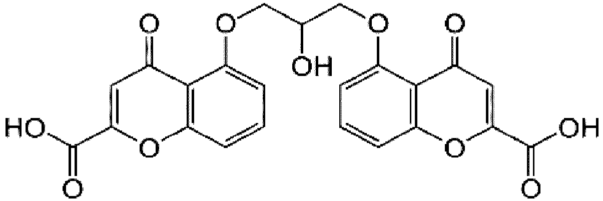

In an exemplary embodiment, the metabolic disease may be any one selected from the group consisting of diabetes, obesity, insulin resistance, and metabolic liver disease. The metabolic liver disease may be non-alcoholic fatty liver disease (NAFLD), and the NAFLD may be any one or more selected from the group consisting of nonalcoholic steatohepatitis (NASH), non-alcoholic fatty liver (NAFL) and NAFLD-associated liver fibrosis, more preferably NASH.

In an exemplary embodiment, prevention or treatment of the metabolic diseases may consist of reducing insulin resistance, reducing body weight and reducing triglyceride accumulation in liver tissue.

In an exemplary embodiment, a pharmaceutically acceptable salt of the cromolyn may be selected from the group consisting of alkali metal salts, alkaline earth metal salts, salts with inorganic acids, salts with organic acids, and salts with acidic amino acids, and most preferably cromolyn sodium.

In an exemplary embodiment, the torsemide and the cromolyn or the pharmaceutically acceptable salts thereof of the present invention may be administered at a dose of 0.01 mg/kg to 1 g/kg of torsemide and cromolyn, respectively.

In an exemplary embodiment, the torsemide and the cromolyn may be administered simultaneously, separately or sequentially.

The present invention includes not only torsemide and cromolyn represented by Chemical Formulas 1 and 2, but also pharmaceutically acceptable salts thereof, and all solvates, hydrates, racemates or stereoisomers, which are able to be prepared therefrom.

The torsemide and the cromolyn represented by Chemical Formulas 1 and 2 of the present invention may be used in the form of pharmaceutically acceptable salts, and the salts are useful with acid additions formed by pharmaceutically acceptable free acids. The acid addition salts are obtained from inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono- and di-carboxylates, phenyl-substituted alkanoates, hydroxy alkanoates and alkanes, dioates, aromatic acids, aliphatic and aromatic sulfonic acids. These pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate or mandelate.

The acid addition salts according to the present invention may be prepared by a conventional method, for example, by dissolving torsemide or cromolyn in an excess aqueous acid solution and precipitating the salt thereof using a water-miscible organic solvent, such as methanol, ethanol, acetone or acetonitrile. In addition, the acid addition salts may also be prepared by evaporating and drying a solvent or excess acid from the mixture or by suction-filtering the precipitated salt. In addition, the pharmaceutically acceptable metal salts may be prepared using bases. An alkali metal salt or an alkaline earth metal salt may be obtained, for example, by dissolving the compound in an excess alkali metal hydroxide or alkaline earth metal hydroxide solution, filtering a non-dissolved compound salt, and then evaporating and drying a filtrate. At this time, as the metal salt, it is pharmaceutically suitable to prepare a sodium, potassium or calcium salt. Further, silver salts corresponding thereto may be obtained by reacting the alkali metal or alkaline earth metal salt with a suitable silver salt (e.g., silver nitrate).

The pharmaceutical composition of the present invention may further include known therapeutic agents for metabolic diseases in addition to the torsemide and/or cromolyn, or salts thereof, as active ingredients, and may be used in combination with other treatments known for the treatment of these diseases.

In the present invention, the term "prevention" means all actions that inhibit or delay the occurrence, spread, and recurrence of metabolic diseases by administration of the pharmaceutical composition according to the present invention. The "treatment" means any action that improves or beneficially alters the symptoms of metabolic diseases by administration of the composition of the present invention. Those skilled in the art to which the present invention pertains will be able to determine the degree of improvement, enhancement and treatment by knowing the exact criteria of a disease for which the composition of the present invention is effective by referring to data presented by the Korean Academy of Medical Sciences, etc.

In the present invention, the term "therapeutically effective dose" used in combination with the active ingredients means an amount effective to prevent or treat a target disease, and the therapeutically effective dose of the composition of the present invention may vary depending on several factors, such as a method of administration, a target site, the condition of a patient. Accordingly, when used in the human body, the dose should be determined as an appropriate amount in consideration of both safety and efficiency. It is also possible to estimate the amount used in humans from the effective dose determined through animal experiments. These matters to be considered when determining the effective dose are described in, for example, Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed. (1990), Mack Publishing Co.

The pharmaceutical composition of the present invention is administered in a pharmaceutically effective dose. As used in the present invention, the term "pharmaceutically effective dose" refers to an amount enough to treat diseases at a reasonable benefit/risk ratio applicable to medical treatment and enough to not cause side effects. An effective dose level may be determined according to a health condition of a patient, a type of metabolic disease, an occurrence cause of metabolic disease, severity, drug activity, sensitivity to a drug, an administration method, an administration time, an administration route and excretion rate, a treatment period, factors including drugs used in combination or concurrently, and other factors well-known in medical fields. The composition of the present invention may be administered as an individual therapeutic agent or in combination with other therapeutic agents, and may be administered sequentially or simultaneously with existing therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present invention may include carriers, diluents, excipients, or a combination of two or more thereof, which are commonly used in biological agents. As used in the present invention, the term "pharmaceutically acceptable" refers to exhibiting non-toxic properties to cells or humans exposed to the composition. The carrier is not particularly limited as long as the carrier is suitable for in vivo delivery of the composition, and may be used by combining, for example, compounds described in Merck Index, 13th ed., Merck & Co. Inc., saline, sterile water, a Ringer's solution, buffered saline, a dextrose solution, a maltodextrin solution, glycerol, ethanol, and at least one of these ingredients, and if necessary, other conventional additives such as an antioxidant, a buffer, and a bacteriostat may be added. In addition, the pharmaceutical composition may be prepared in injectable formulations such as an aqueous solution, a suspension, and an emulsion, pills, capsules, granules, or tablets by further adding a diluent, a dispersant, a surfactant, a binder, and a lubricant. Furthermore, the pharmaceutical composition may be prepared preferably according to each disease or ingredient using as a suitable method in the art or a method disclosed in Remington's Pharmaceutical Science (Mack Publishing Company, Easton PA, 18th, 1990).

In an exemplary embodiment, the pharmaceutical composition may be one or more formulations selected from the group consisting of oral formulations, external preparations, suppositories, sterile injections and sprays, and more preferably oral or injection formulations.

As used in the present invention, the term "administration" means providing a predetermined substance to a subject or patient by any suitable method, and the pharmaceutical composition may be administered parenterally (applied as an injectable formulation, e.g., intravenously, subcutaneously, intraperitoneally or topically) or orally according to a desired method, and the dose range may vary depending on the body weight, age, sex, and health condition of a patient, a diet, an administration time, an administration method, an excretion rate, and the severity of a disease. Liquid formulations for oral administration of the composition of the present invention correspond to suspensions, internal solutions, emulsions, syrups, etc., and may include various excipients, such as wetting agents, sweeteners, fragrances, preservatives, and the like in addition to water and liquid paraffin, which are commonly used simple diluents. Formulations for parenteral administration include sterilized aqueous solutions, non-aqueous solvents, suspensions, emulsions, lyophilized agents, suppositories, and the like. The pharmaceutical composition of the present invention may also be administered by any device capable of transferring an active substance to a target cell. Preferred methods of administration and formulations are intravenous injections, subcutaneous injections, intradermal injections, intramuscular injections, drop injections, or the like. The injections may be prepared by using aqueous solvents such as a physiological saline solution and a ringer solution, and non-aqueous solvents such as vegetable oils, higher fatty acid esters (e.g., ethyl oleate, etc.), and alcohols (e.g., ethanol, benzyl alcohol, propylene glycol, glycerin, or the like). The injections may include pharmaceutical carriers, such as a stabilizer for the prevention of degeneration (e.g., ascorbic acid, sodium hydrogen sulfite, sodium pyrosulfite, BHA, tocopherol, EDTA, etc.), an emulsifier, a buffer for pH control, and a preservative to inhibit microbial growth (e.g., phenyl mercury nitrate, thimerosal, benzalkonium chloride, phenol, cresol, benzyl alcohol, etc.).

As used in the present invention, the term "subject" refers to all animals including monkeys, cows, horses, sheep, pigs, chickens, turkeys, quails, cats, dogs, mice, rats, rabbits or guinea pigs including humans who have developed or may develop the metabolic diseases, and the pharmaceutical composition of the present invention may be administered to a subject to effectively prevent or treat the diseases. The pharmaceutical composition of the present invention may be administered in combination with existing therapeutic agents.

The pharmaceutical composition of the present invention may further include pharmaceutically acceptable additives. At this time, the pharmaceutically acceptable additives may use starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, talc and the like. The pharmaceutically acceptable additive according to the present invention is preferably included in an amount of 0.1 part by weight to 90 parts by weight based on the composition, but is not limited thereto.

In an aspect, the present invention provides a health functional food for preventing and improving metabolic diseases containing torsemide and cromolyn, or pharmaceutically acceptable salts thereof.

In an exemplary embodiment, the torsemide may be a compound represented by Chemical Formula 1 below, and the cromolyn may be a compound represented by Chemical Formula 2 below: 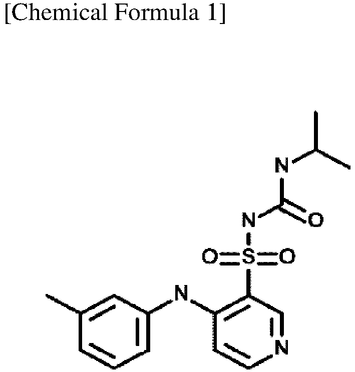 ; and

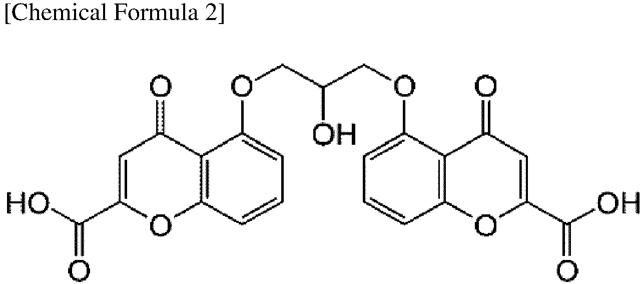

In an exemplary embodiment, the metabolic disease may be any one selected from the group consisting of diabetes, obesity, insulin resistance, and metabolic liver disease. The metabolic liver disease may be non-alcoholic fatty liver disease (NAFLD), and the NAFLD may be any one or more selected from the group consisting of nonalcoholic steatohepatitis (NASH), non-alcoholic fatty liver (NAFL) and NAFLD-associated liver fibrosis, more preferably NASH.

In an exemplary embodiment, prevention or treatment of the metabolic diseases may consist of reducing insulin resistance, reducing body weight and reducing triglyceride accumulation in liver tissue.

In an exemplary embodiment, a pharmaceutically acceptable salt of the cromolyn may be selected from the group consisting of alkali metal salts, alkaline earth metal salts, salts with inorganic acids, salts with organic acids, and salts with acidic amino acids, and most preferably cromolyn sodium.

In an exemplary embodiment, the torsemide and the cromolyn or the pharmaceutically acceptable salts thereof of the present invention may be administered at a dose of 0.01 mg/kg to 1 g/kg of torsemide and cromolyn, respectively.

In an exemplary embodiment, the torsemide and the cromolyn may be administered simultaneously, separately or sequentially.

When the composition of the present invention is used as the food composition, the composition may be added as it is or used with other foods or food ingredients, and may be appropriately used according to a general method. The composition may include food acceptable supplement additives in addition to the active ingredients, and the mixing amount of the active ingredients may be appropriately determined depending on the purpose of use (prevention, health or therapeutic treatment).

The term "food supplement additive" used in the present invention means a component that may be supplementally added to food, and may be appropriately selected and used by those skilled in the art as being added to prepare a health functional food of each formulation. Examples of the food supplement additives include various nutrients, vitamins, minerals (electrolytes), flavors such as synthetic and natural flavors, colorants and fillers, pectic acid and salts thereof, alginic acid and salts thereof, organic acids, protective colloidal thickeners, pH adjusters, stabilizers, preservatives, glycerin, alcohols, carbonation agents used in carbonated drinks, and the like, but the types of food supplement additives of the present invention are not limited by the examples.

The food composition of the present invention may include a health functional food. The term "health functional food" used in the present invention refers to food prepared and processed in the form of tablets, capsules, powders, granules, liquids and pills using raw materials or ingredients having functionalities useful to the human body. Here, the 'functionality' means regulating nutrients to the structure and function of the human body or obtaining effects useful for health applications such as physiological action. The health functional food of the present invention is able to be prepared by methods to be commonly used in the art and may be prepared by adding raw materials and ingredients which are commonly added in the art in preparation. In addition, the formulations of the health functional food may also be prepared with any formulation recognized as a health functional food without limitation. The food composition of the present invention may be prepared in various types of formulations, and unlike general drugs, the food composition has an advantage that there is no side effect that may occur when taking a long-term use of the drug using the food as a raw material, and has excellent portability, but the health functional food of the present invention may be taken as supplements to enhance the effects of the therapeutic agents for metabolic diseases.

In addition, there is no limitation in a type of health food in which the composition of the present invention may be used. In addition, the composition including the composition of the present invention as the active ingredient may be prepared by mixing known additives with other suitable auxiliary ingredients that may be contained in the health functional food according to the selection of those skilled in the art. Examples of foods to be added include meat, sausage, bread, chocolate, candy, snacks, confectionery, pizza, ramen, other noodles, gum, dairy products including ice cream, various soups, beverages, tea, drinks, alcoholic beverages, vitamin complexes and the like, and may be prepared by adding extract, tea, jelly, juice, and the like prepared by using the extract according to the present invention as a main ingredient.

In an aspect, the present invention provides a method for treating metabolic diseases including administering torsemide and cromolyn, or pharmaceutically acceptable salts thereof in a pharmaceutically effective dose to a subject suffering from metabolic diseases.

In an aspect, the present invention provides a use of torsemide and cromolyn, or pharmaceutically acceptable salts thereof, for use in the preparation of a pharmaceutical composition for preventing or treating metabolic diseases.

### [Modes of the Invention]

Hereinafter, the present invention will be described in more detail with reference to the following Examples. However, the following Examples are only intended to embody the contents of the present invention, and the present invention is not limited thereto.

### Example 1. Confirmation of body weight change in nonalcoholic steatohepatitis animal model

A nonalcoholic steatohepatitis (NASH) animal model was prepared, and in order to confirm the effect of a drug of the present invention on the NASH animal model, nonalcoholic steatohepatitis (NASH) was caused using a high fat diet model in which high-fat feed with a fat content of 60% was taken for 12 weeks. Specifically, 21 6-week-old ICR mice were preliminarily bred for 7 days before the start of the experiment with general feed to adapt a breeding environment, and divided into a control group (n = 3, normal fat diet (NFD) taken with feed with 10% fat content), a high-fat diet control group (n = 9, high fat diet, HFD), a high-fat diet experimental group (n = 6, cromolyn treat), and a high-fat diet experimental group (n = 6, cromolyn + torsemide treat) (NASH-induced group). A total breeding period was 13 weeks, and the experimental diet and drinking water were freely taken, and all experimental diets were stored in refrigeration during the breeding period. The drug administration was conducted for 6 weeks from week 6 after starting the high-fat diet, and in the control group, phosphate buffered saline (PBS) was administered, and in the experimental group, 10 mg/kg of cromolyn sodium or 10 mg/kg of cromolyn + 10 mg/kg of torsemide was administered intraperitoneally three times a week. The body weights of the mice in the group were measured at regular times once a week during the experimental period to confirm changes in body weight.

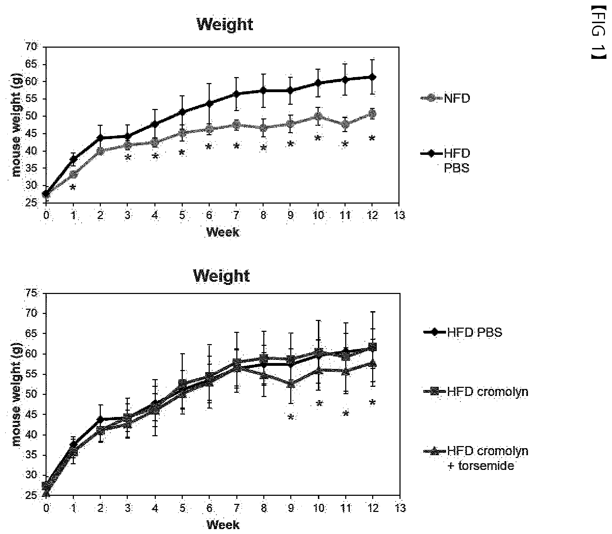

As a result, it was confirmed that after 12 weeks, in the high-fat diet experimental group (HFD), the average body weight was 61.3 g, which was significantly higher than that of the normal diet group (NFD) having an average body weight of 50.8 g, so that the NASH animal model was well established (FIG. 1A). In addition, a high-fat diet experimental group (HFD cromolyn) administered with cromolyn showed no significant change in body weight compared to a high-fat diet control group (HFD PBS). On the contrary, in the case of the high-fat diet experimental group (HFD cromolyn + torsemide) administered in combination with cromolyn and torsemide, the body weight decreased significantly from 9 weeks of age, and at 12 weeks, the body weight was 57.8 g, which was a statistically significant decrease in body weight compared to the average body weight of the high-fat diet experimental group (HFD) (*p-value < 0.05) (FIG. 1B).

### Example 2. Confirmation of insulin resistance in NASH animal model

To confirm insulin resistance in the NASH animal model administered with cromolyn and/or torsemide, changes in blood sugar levels of mice for each group were confirmed at Week 13 of the experiment. Specifically, after diet and drug administration for 13 weeks as in Example 1, the experimental animals were fasted for 16 hours and fasted blood was collected from the peripheral tail blood vessels. In addition, 1.5 mg/kg of a glucose solution (Sigma Aldrich, St. Louis, MO, USA) was administered intraperitoneally to each group and then blood was collected from the tail vein at 15, 30, 45, 60, 90, and 120 minutes, respectively, and changes in blood sugar concentration were measured.

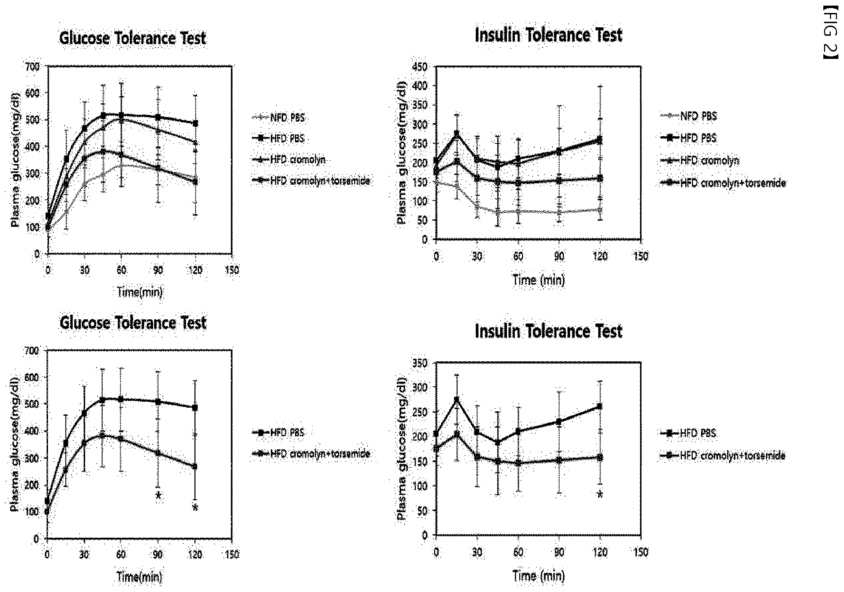

As a result of a glucose tolerance test (GTT), at 90 minutes, the average fasting blood sugar level (mg/dl) of the control group (HFD PBS) was 509.6 mg/dl, and the average fasting blood sugar level (mg/dl) of the experimental group (cromolyn + torsemide treat) was 319.4 mg/dl, and at 120 minutes, the average fasting blood sugar levels (mg/dl) were 486 mg/dl and 267.4 mg/dl, respectively, so that the blood sugar levels were significantly reduced by combined treatment with cromolyn and torsemide (FIG. 2). In addition, as a result of an insulin tolerance test (ITT), at 120 minutes, the blood sugar level of the control group (HFD PBS) was 260 mg/dl and the blood sugar level of the experimental group (cromolyn + torsemide treat) was 158.6 mg/dl, so that the blood sugar level in the experimental group was shown lower. Accordingly, it was confirmed that the control group had higher insulin resistance than the experimental group, and in the experimental group, insulin serving to lower blood sugar was working well, and thus, insulin resistance was significantly lowered (*p-value < 0.05) (FIG. 2).

### Example 3. Confirmation of triglycerides in liver tissue

To confirm changes in the degree of fat accumulation in the NASH animal model by administration of cromolyn and/or torsemide, Oil Red O staining analysis was performed. Specifically, after diet and drug administration for 13 weeks as in Example 1, experimental animals for each group were sacrificed, livers were extracted, and tissue blocks in a cryo state were sectioned at a thickness of 12 µm. An Oil RED O working solution (oil red O: D.W. 3 : 2) was treated on the sectioned tissue and incubated at room temperature for 5 minutes. Thereafter, the tissue was washed under running water for 20 minutes, and the tissue was mounted on a cover slip using a water-soluble mounting medium. Then, the tissue was incubated at room temperature for 10 minutes and observed under a microscope to confirm changes in the accumulation degree of triglycerides in liver tissue due to drug treatment.

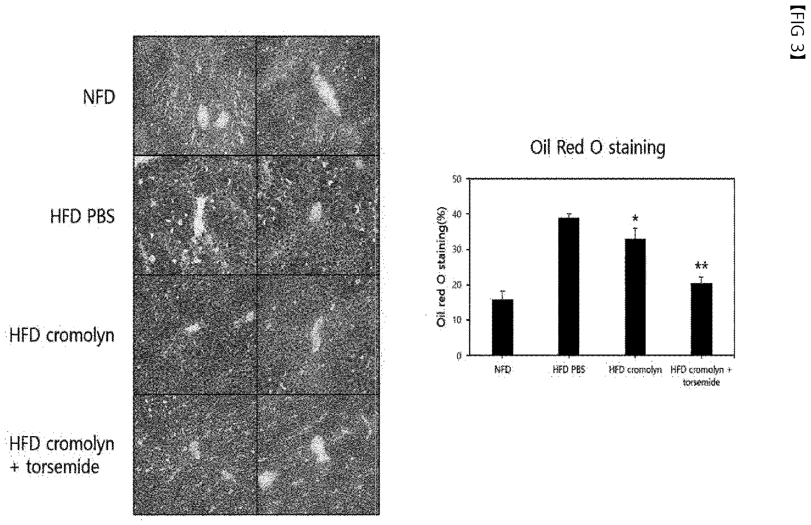

As a result, it was found that the triglycerides in liver tissue, which increased in the HFD PBS group compared to the control group (NFD, HFD PBS), were relatively decreased in the experimental group (HFD cromolyn, HFD cromolyn + torsemide). In particular, the triglycerides were significantly decreased in the group treated in combination with cromolyn and torsemide (*p-value < 0.05) (FIG. 3).

## Claims

1. A pharmaceutical composition for preventing or treating metabolic diseases comprising torsemide and cromolyn, or pharmaceutically acceptable salts thereof, as active ingredients.

2. The pharmaceutical composition for preventing or treating metabolic diseases of claim 1, wherein the torsemide is a compound represented by Chemical Formula 1 below:

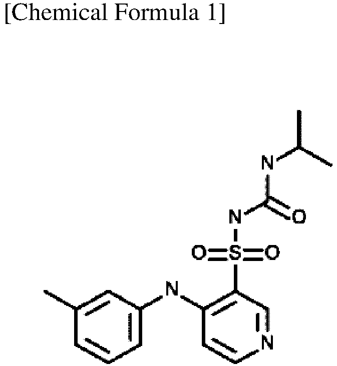

3. The pharmaceutical composition for preventing or treating metabolic diseases of claim 1, wherein the cromolyn is a compound represented by Chemical Formula 2 below:

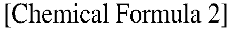

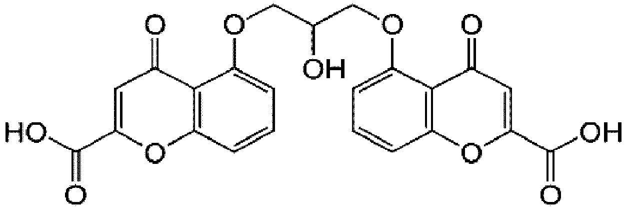

4. The pharmaceutical composition for preventing or treating metabolic diseases of claim 1, wherein the metabolic diseases are any one selected from the group consisting of diabetes, obesity, insulin resistance, and metabolic liver disease.

5. The pharmaceutical composition for preventing or treating metabolic diseases of claim 4, wherein the metabolic liver disease is non-alcoholic fatty liver disease (NAFLD).

6. The pharmaceutical composition for preventing or treating metabolic diseases of claim 5, wherein the non-alcoholic fatty liver disease is any one or more selected from the group consisting of nonalcoholic steatohepatitis (NASH), non-alcoholic fatty liver (NAFL), and NAFLD-associated liver fibrosis.

7. The pharmaceutical composition for preventing or treating metabolic diseases of claim 1, wherein the insulin resistance is reduced.

8. The pharmaceutical composition for preventing or treating metabolic diseases of claim 1, wherein the body weight is reduced.

9. The pharmaceutical composition for preventing or treating metabolic diseases of claim 1, wherein the triglycerid accumulation in liver tissue is reduced.

10. The pharmaceutical composition for preventing or treating metabolic diseases of claim 1, wherein the salt of cromolyn is cromolyn sodium.

11. A health functional food for preventing or improving metabolic diseases containing torsemide and cromolyn, or pharmaceutically acceptable salts thereof.

12. The health functional food for preventing or improving metabolic diseases of claim 11, wherein the torsemide is a compound represented by Chemical Formula 1 below:

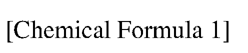

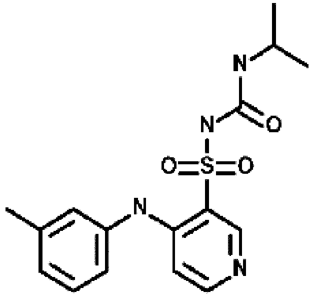

13. The health functional food for preventing or improving metabolic diseases of claim 11, wherein the cromolyn is a compound represented by Chemical Formula 2 below:

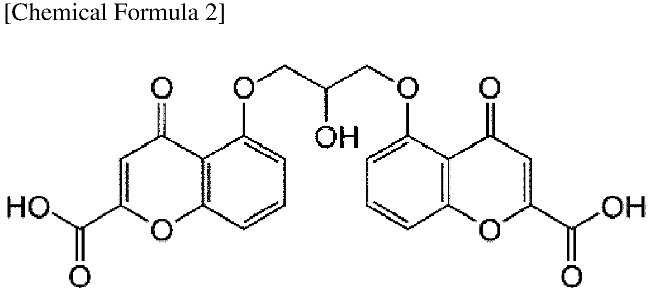

14. The health functional food for preventing or improving metabolic diseases of claim 11, wherein the metabolic diseases are any one selected from the group consisting of diabetes, obesity, insulin resistance, and metabolic liver disease.

15. The health functional food for preventing or improving metabolic diseases of claim 14, wherein the metabolic liver disease is non-alcoholic fatty liver disease (NAFLD).

16. The health functional food for preventing or improving metabolic diseases of claim 15, wherein the non-alcoholic fatty liver disease is any one or more selected from the group consisting of nonalcoholic steatohepatitis (NASH), non-alcoholic fatty liver (NAFL), and NAFLD-associated liver fibrosis.

17. A method for treating metabolic diseases comprising administering torsemide and cromolyn, or pharmaceutically acceptable salts thereof in a pharmaceutically effective dose to a subject suffering from metabolic diseases.

18. A use of torsemide and cromolyn, or pharmaceutically acceptable salts thereof, for use in the preparation of a pharmaceutical composition for preventing or treating metabolic diseases.
